# EUROPEAN PATENT APPLICATION

(11) **EP 4 659 676 A1**
(43) Date of publication of application: **10.12.2025**
(21) Application number: 25180595.8
(22) Date of filing: 03.06.2025
(51) Int. Cl.: A61B 6/00

(54) **ELECTRIC TRAVELING UNIT AND RADIOGRAPHY SYSTEM**

(30) Priority: 06.06.2024 JP 2024092507
(71) Applicant: FUJIFILM Corporation, Tokyo 106-8620 (JP)
(72) Inventor: HORIUCHI, Hisatsugu, Kanagawa, 258-8538 (JP); MORI, Kyohei, Kanagawa, 258-8538 (JP); TANEICHI, Tatsuya, Kanagawa, 258-8538 (JP); NISHINO, Naoyuki, Kanagawa, 258-8538 (JP); KOBAYASHI, Takeyasu, Kanagawa, 258-8538 (JP)
(74) Representative: Dehns Germany Partnerschaft mbB

(57) **Abstract**

An electric traveling unit (30) that enables electric traveling of an imaging unit (11) that includes a traveling mechanism traveling on a floor (FL) manually and a radiation source or a radiation detector used for radiography, in which the electric traveling unit (30) separably combines with the imaging unit (11), and reduces a traveling resistance of the traveling mechanism in a combined state than in a non-combined state.

## Description

### BACKGROUND OF THE INVENTION

### 1. Field of the Invention

The present disclosure relates to an electric traveling unit and a radiography system.

### 2. Description of the Related Art

JP5211405B discloses a mobile X-ray imaging apparatus comprising a connecting unit that attachably and detachably connects an X-ray imaging unit and a main body unit, in which the X-ray imaging unit is separated from the main body unit to perform X-ray imaging, and the X-ray imaging unit and the main body unit are connected by the connecting unit to be capable of being integrally transported. The X-ray imaging unit is capable of manual traveling in which a user causes the X-ray imaging unit to manually travel on a floor, and the main body unit is capable of electric traveling in which the main body unit is caused to electrically travel on the floor. The main body unit pulls the X-ray imaging unit in a state of being connected to the X-ray imaging unit by the connecting unit.

### SUMMARY OF THE INVENTION

For an imaging unit such as the X-ray imaging unit described in JP5211405B, usability is more favorable in a case where the imaging unit can perform both the manual traveling and the electric traveling.

However, the technique described in JP5211405B has the following problems. That is, a traveling mechanism that causes the imaging unit to travel manually is often provided with a brake so that the imaging unit is not moved inadvertently. The brake is grounded on the floor in an operation state or restricts a rotation of wheels to increase a traveling resistance such as a frictional force between the traveling mechanism and the floor, and restricts a movement of the imaging unit by increasing the traveling resistance. It is impossible to pull the imaging unit in a state where the traveling resistance is large, such as a case where the brake is applied. In addition, in a case where a weight of the imaging unit is heavy, a traveling resistance such as a frictional force between the floor and the wheels is also large, and in a case where a driving force of the main body unit is small, the main body unit may not be able to be pulled.

The technology of the present disclosure provides an electric traveling unit and a radiography system that enable electric traveling of an imaging unit even in a case in which a traveling resistance of the imaging unit is large.

An electric traveling unit according to the technology of the present disclosure is an electric traveling unit that enables electric traveling of an imaging unit that includes a traveling mechanism traveling on a floor manually and a radiation source or a radiation detector used for radiography, in which the electric traveling unit separably combines with the imaging unit, and reduces a traveling resistance of the traveling mechanism in a combined state than in a non-combined state.

In the above aspect, a load may be generated above the imaging unit in a vertical direction in the combined state.

In the above aspect, the electric traveling unit may comprise a load generating mechanism that generates the load.

In the above aspect, the load generating mechanism may include a support portion that supports the imaging unit while maintaining a posture of the imaging unit in the non-combined state, and generate the load by raising and lowering the support portion.

In the above aspect, the load may be a load that lifts at least a part of the imaging unit from the floor.

In the above aspect, the imaging unit may be provided with a brake that increases the traveling resistance, and the load may be a load that reduces the traveling resistance caused by the brake.

In the above aspect, a portion of the imaging unit that is grounded on the floor in the non-combined state may be maintained in a grounded state even in a state where the load is generated.

In the above aspect, the electric traveling unit may mechanically or magnetically bond to the imaging unit.

In the above aspect, at least a part of the electric traveling unit may enter a projection region of the imaging unit in a case where the floor is used as a projection surface, in the combined state.

In the above aspect, the electric traveling unit may comprise a plurality of wheels in which at least one wheel is a driving wheel that rotates electrically, in which a wheel spacing between the plurality of wheels may be narrower than a wheel spacing of the imaging unit.

In the above aspect, an entire electric traveling unit may enter the projection region of the imaging unit in a case where the floor is used as the projection surface, in the combined state.

In the above aspect, the electric traveling unit may have a function of automatically combining with the imaging unit by a combination instruction in a state of being separated from the imaging unit.

In the above aspect, the electric traveling unit may detect a position of the imaging unit and move to the detected position.

In the above aspect, the electric traveling unit may have a function of automatically moving to a designated position.

In the above aspect, the electric traveling unit may indirectly receive an operation instruction including a movement instruction via the imaging unit.

In the above aspect, the electric traveling unit may comprise a wireless communication unit that performs wireless communication with the imaging unit.

In the above aspect, the electric traveling unit may receive the movement instruction as the operation instruction through an operating part of a type in which an output of an operation signal is continued while the operating part is being operated and the output of the operation signal is stopped in a case where the operation is stopped.

In the above aspect, the electric traveling unit may comprise a battery that functions as an auxiliary battery of the imaging unit in the combined state.

A radiography system according to the technology of the present disclosure comprises at least one imaging unit that includes a traveling mechanism traveling on a floor manually and a radiation source or a radiation detector used for radiography, and an electric traveling unit that enables electric traveling of the imaging unit, the electric traveling unit separably combining with the imaging unit and reducing a traveling resistance of the traveling mechanism in a combined state than in a non-combined state.

According to the technology of the present disclosure, even in a case where the traveling resistance of the imaging unit is large, the imaging unit can travel electrically.

### BRIEF DESCRIPTION OF THE DRAWINGS

Fig. 1 is a diagram showing an example of a schematic configuration of a radiography system.
Fig. 2 is a diagram showing an example of a procedure in which an imaging unit and an electric traveling unit combine with each other.
Fig. 3 is a diagram showing an example of a load generating mechanism.
Fig. 4 is a diagram showing an example of a size of the electric traveling unit.
Fig. 5 is a block diagram showing an example of a functional configuration of the electric traveling unit.
Fig. 6 is a diagram showing an example of a controller.
Fig. 7 is a diagram showing an example of traveling control of the electric traveling unit.
Fig. 8 is an example of a flowchart showing a processing procedure of the electric traveling unit.
Fig. 9 is a diagram showing a use example of the electric traveling unit.
Fig. 10 is a diagram showing another example of the use example of the electric traveling unit.
Fig. 11 is a diagram showing an example in which an imaging menu and a registered position are associated with each other.
Fig. 12 is a diagram showing a combined state in which a part of the electric traveling unit is exposed.
Fig. 13 is a diagram showing an example of detection of the electric traveling unit.
Fig. 14 is a diagram showing an example in which a grounded state is maintained in the combined state.
Fig. 15 is a diagram showing an example in which a load is generated without using a mechanism.
Fig. 16 is a diagram showing an example of a guide unit.
Fig. 17 is a diagram showing an example of an auxiliary battery.

### DESCRIPTION OF THE PREFERRED EMBODIMENTS

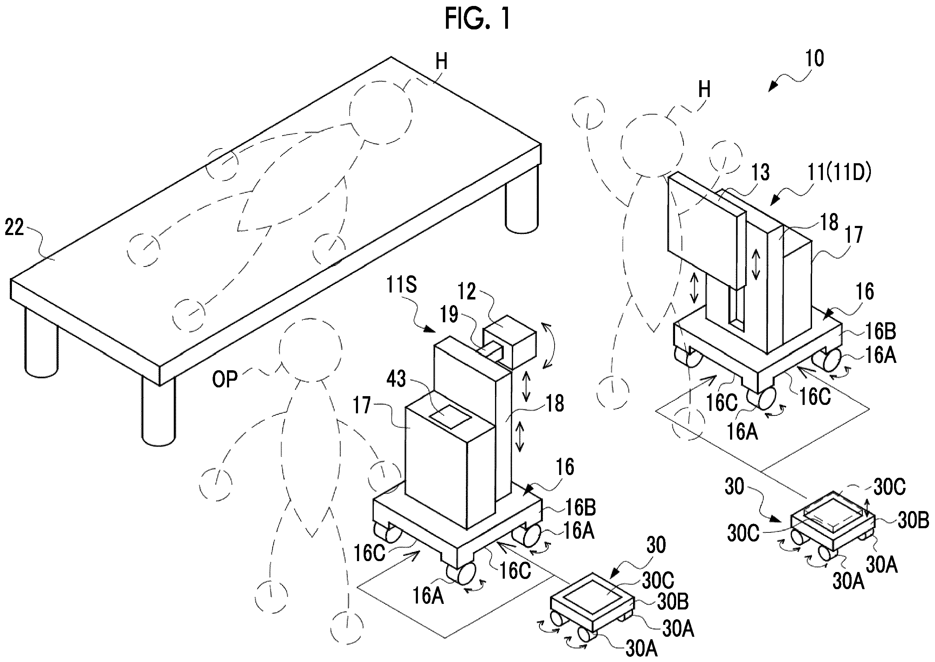

Fig. 1 is a schematic diagram showing an example of the configuration of a radiography system 10 that performs radiography of a subject H. The radiography system 10 comprises a radiation source unit 11S and a panel unit 11D. These are deployed in a radiography room as an example and are operated by an operator OP such as a radiology technician. The radiation source unit 11S has an irradiation unit 12. The irradiation unit 12 is an example of a "radiation source" according to the technology of the present disclosure. The irradiation unit 12 includes an X-ray tube that generates X-rays, an irradiation field limiter that limits an irradiation range of the X-rays, and the like. The radiation source unit 11S is also provided with a high-voltage generator that generates a high voltage to be supplied to the X-ray tube, and the like.

The panel unit 11D includes a detection panel 13 which is an example of a radiation detection panel. The detection panel 13 is, for example, a flat panel detector that has a detection surface on which X-rays are detected and pixels that output an electric signal according to an incidence amount of the X-rays are two-dimensionally arranged. The detection panel 13 is an example of a "radiation detector" according to the technology of the present disclosure.

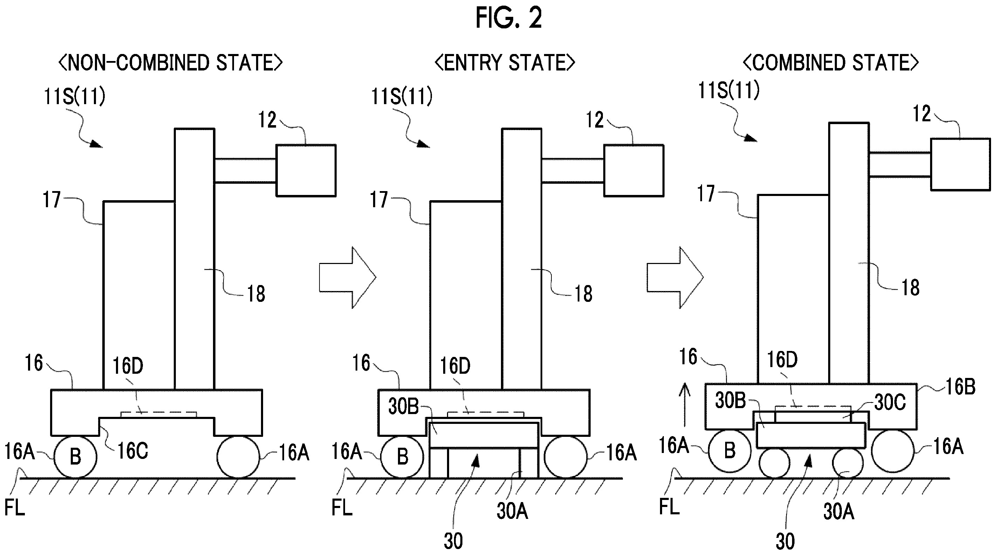

The radiography system 10 irradiates the subject H with X-rays, which are an example of radiation, from the irradiation unit 12 and detects the X-rays transmitted through the subject H with the detection panel 13 to capture an X-ray image of the subject H. The radiation source unit 11S and the panel unit 11D each have a carriage unit 16 having wheels 16A. The carriage unit 16 includes, for example, a base unit 16B that is a body part having a rectangular planar shape. The wheels 16A are provided at four corners of the base unit 16B, and the carriage unit 16 is of a four-wheel type. Each wheel 16A is, for example, a revolution type that revolves around a revolution axis extending in a height direction (also referred to as a vertical direction) orthogonal to a rotation axis in a case of traveling and rotating. The carriage unit 16 can manually travel on a floor FL (see Fig. 2 and the like). Therefore, the operator OP can move installation locations of the radiation source unit 11S and the panel unit 11D by manually causing the carriage unit 16 to travel. The carriage unit 16 is an example of a "traveling mechanism" that travels on the floor FL manually.

The radiation source unit 11S has a body part 17 and a movable mechanism that changes the height of the irradiation unit 12. The movable mechanism is composed of a movable unit 18 of which a height changes with respect to the body part 17 and an arm 19 of which a height changes with respect to the movable unit 18. The irradiation unit 12 is provided at the distal end of the arm 19. The irradiation unit 12 is provided at the free end of the arm 19. In addition, although not shown, the arm 19 is provided with a swing mechanism that changes the irradiation direction by tilting the irradiation unit 12. Various electrical components are incorporated in the body part 17, and an operation panel 43 is provided on the upper surface thereof.

The panel unit 11D also has the body part 17 and a movable mechanism that changes the height of the detection panel 13, similarly to the radiation source unit 11S. The movable mechanism of the panel unit 11D is also composed of the movable unit 18 of which the height changes with respect to the body part 17 and the arm (not shown) of which the height changes with respect to the movable unit 18, and is the same as the movable mechanism of the radiation source unit 11S. The detection panel 13 is provided at the free end of the arm. Various electrical components are also incorporated in the body part 17 and the operation panel 43 is provided on the upper surface thereof, similarly to the body part 17 of the radiation source unit 11S.

The body part 17, the movable unit 18, and the arm 19 of the radiation source unit 11S and the body part 17, the movable unit 18, and the arm of the panel unit 11D are, of course, different in some parts such as incorporated components and shapes. However, in the technology of the present disclosure, the difference between the radiation source unit 11S and the panel unit 11D is not a main point, and thus both are represented by the same reference sign. Both the radiation source unit 11S and the panel unit 11D are examples of an "imaging unit" according to the technology of the present disclosure. In the following, in a case where it is not necessary to distinguish between the radiation source unit 11S and the panel unit 11D, both are referred to as an imaging unit 11.

As shown in Fig. 1, the radiation source unit 11S and the panel unit 11D are disposed to face each other with the subject H interposed therebetween in a case where the subject H is imaged in an upright posture, as an example. In addition, in a case in which imaging is performed using a decubitus imaging table 22 on which the subject H can be placed in a decubitus posture, for example, a portable detection panel (not shown) called an electronic cassette and the radiation source unit 11S are used in combination. Of course, the detection panel may be incorporated in the decubitus imaging table 22, and in this case, the incorporated detection panel may be used.

In addition, the radiography system 10 comprises an electric traveling unit 30 in addition to each imaging unit 11 of the radiation source unit 11S and the panel unit 11D. The electric traveling unit 30 separably combines with the imaging unit 11 that can manually travel, and enables the electric traveling of the imaging unit 11.

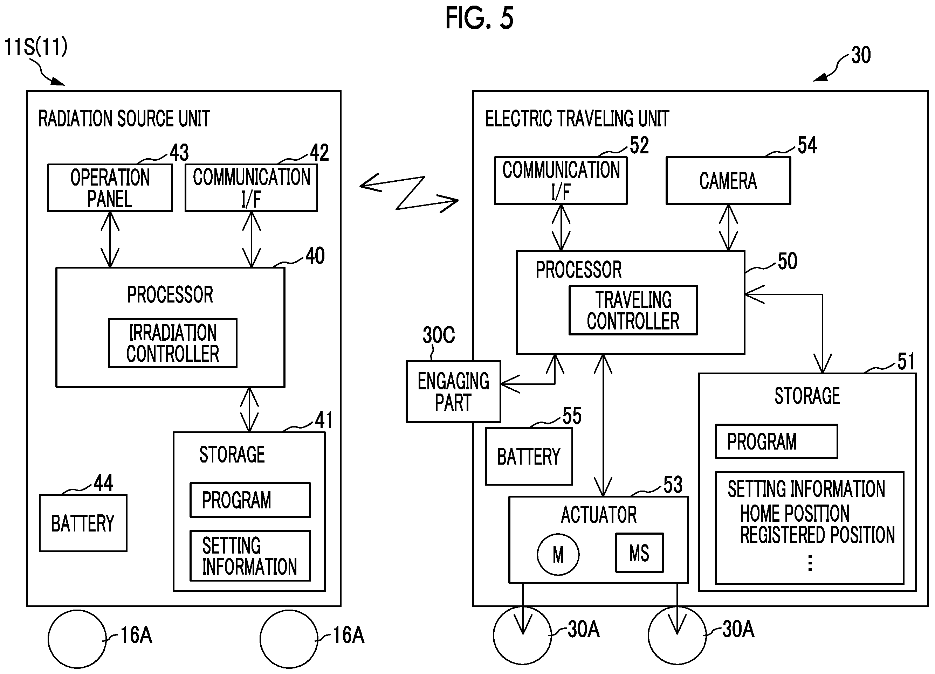

The electric traveling unit 30 has a body part 30B and wheels 30A that function as a traveling mechanism that travels on the floor FL. An electric circuit for electric traveling and electrical components such as an actuator 53 (see Fig. 5) are incorporated in the body part 30B. As an example, the body part 30B has a rectangular planar shape as in the carriage unit 16. The wheels 30A are provided at four corners of the body part 30B, and the electric traveling unit 30 is of a four-wheel type. Each wheel 30A is, for example, a revolution type that revolves around a revolution axis extending in a height direction (also referred to as a vertical direction) orthogonal to a rotation axis in a case of traveling and rotating, and all the wheels 30A function as steering wheels. In addition, as an example, all of the wheels 30A are driving wheels that are electrically rotated. Of course, two of the four wheels 30A may be used as steering wheels, and the remaining two may be used as driving wheels.

The electric traveling unit 30 enters between the bottom portion of the base unit 16B of the imaging unit 11 and the floor FL and combines with the imaging unit 11. The base unit 16B of the imaging unit 11 is provided with an entry port 16C for receiving the electric traveling unit 30 between the two wheels 16A. In addition, the body part 30B of the electric traveling unit 30 includes an engaging part 30C that is freely raised and lowered in the height direction. The engaging part 30C is at a lowering position in a normal state, and does not protrude from the upper surface of the body part 30B at the lowering position. In a case where the engaging part 30C rises from the lowering position as indicated by a dotted line, a part of the engaging part 30C protrudes from the upper surface of the body part 30B. The engaging part 30C has, for example, a flat plate shape with a rectangular planar shape, and the upper surface thereof comes into contact with the bottom portion of the base unit 16B.

As shown in Fig. 2 as an example, in a state in which the imaging unit 11 does not combine with the electric traveling unit 30 (hereinafter, referred to as a non-combined state), there is a space in which the electric traveling unit 30 can be received between the base unit 16B and the floor surface. The electric traveling unit 30 enters from the entry port 16C below the base unit 16B of the imaging unit 11 in the non-combined state. In the entry state, the engaging part 30C of the electric traveling unit 30 is still at the lowering position. In the entry state, in a case where the engaging part 30C rises, a part of the engaging part 30C enters a recessed portion 16D formed in the bottom portion of the base unit 16B of the imaging unit 11, and the recessed portion 16D and the engaging part 30C are engaged with each other. Due to the engagement, the electric traveling unit 30 and the imaging unit 11 mechanically bond to each other, and the electric traveling unit 30 and the imaging unit 11 are in a combined state (hereinafter, referred to as a combined state). Since the imaging unit 11 is integrated with the electric traveling unit 30 in the combined state, the imaging unit 11 is also moved together with the electric traveling unit 30 in a case where the electric traveling unit 30 travels. Accordingly, the imaging unit 11 can perform electric traveling.

In addition, in the combined state, the engaging part 30C presses the bottom portion of the base unit 16B from below to generate a load above the imaging unit 11 in the vertical direction. The engaging part 30C is an example of a "load generating mechanism" according to the technology of the present disclosure. In the present example, as shown in Fig. 2, in the combined state, the imaging unit 11 is lifted by the engaging part 30C to the extent that the wheels 16A are lifted from the floor FL. In a case where the wheels 16A are grounded on the floor FL, a frictional force corresponding to the weight of the imaging unit 11 is generated between the wheels 16A of the imaging unit 11 and the floor FL. Since the engaging part 30C generates a load upward in the vertical direction, at least a part of the weight of the imaging unit 11 is reduced, and thus the frictional force between the wheels 16A and the floor FL is reduced. As described above, in the combined state, the electric traveling unit 30 reduces the traveling resistance of the traveling mechanism including the wheels 16A of the imaging unit 11 than in the non-combined state.

In Fig. 2, in the entry state, a state is shown in which the electric traveling unit 30 enters through the side entry port 16C of the imaging unit 11. Therefore, the orientation of the wheels 30A is inclined by 90° with respect to the orientation of the wheels 16A of the imaging unit 11. On the other hand, in the combined state, the wheels 30A revolve by 90° and are in a state of facing the same direction as the orientation of the wheels 16A of the imaging unit 11.

The imaging unit 11 is often provided with a brake that increases the traveling resistance of the wheels 16A, for example, so that the imaging unit 11 is not moved inadvertently at the installation location. In Fig. 2, a reference sign "B" shown in some wheels 16A of the imaging unit 11 indicates that the brake is applied to the wheels 16A and the rotation is restricted. The brake of the present example is a brake that increases the rolling resistance of the wheels 16A as the traveling resistance. In the combined state, the wheels 16A lift from the floor FL. Therefore, even in a case where the brake is applied to the wheels 16A, the brake is not the traveling resistance. As the brake, there is also a type of brake that is provided independently of the wheels 16A, is grounded on the floor FL to generate a frictional force, and increases the traveling resistance. Even in this case, the frictional force between the brake and the floor FL is reduced by the generation of the load upward in the vertical direction by the engaging part 30C, so that the traveling resistance is reduced.

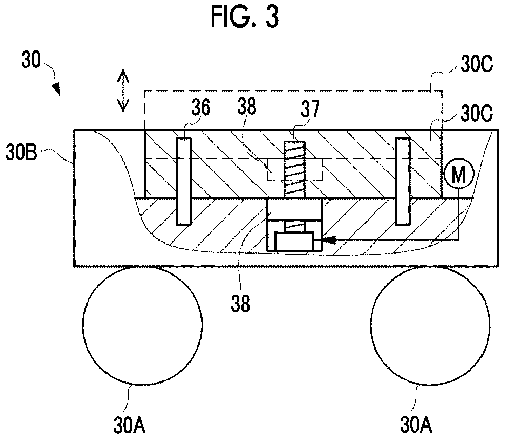

Fig. 3 shows a specific example of the load generating mechanism including the engaging part 30C. The engaging part 30C is attached to the body part 30B to be freely raised and lowered via an elevating guide 36 fixed to the body part 30B. The elevating guide 36 has a rod shape extending in the vertical direction, and is provided at four corners of the engaging part 30C in a case of being viewed in a plan view. The elevating guide 36 guides the engaging part 30C such that the engaging part 30C is raised and lowered while maintaining the posture. The engaging part 30C is raised and lowered by a ball screw 37. One end of the ball screw 37 is fixed to the body part 30B. A slider 38 that slides in the axial direction of the ball screw 37 is fixed to the engaging part 30C. The ball screw 37 is driven by a motor M. In a case where the ball screw 37 rotates about the axis, the slider 38 is raised and lowered, and the engaging part 30C is raised and lowered accordingly. The load generating mechanism of the present example is an example, and may be an elevating mechanism having a pantograph type link mechanism, or the like in addition to using the ball screw 37.

In addition, the engaging part 30C of the present example supports the imaging unit 11 by the upper surface of the engaging part 30C coming into contact with the bottom portion of the base unit 16B. The engaging part 30C is an example of a "support portion" according to the technology of the present disclosure. As shown in Fig. 3 as an example, the engaging part 30C is raised and lowered along the vertical direction in a state where the posture is maintained. Therefore, the imaging unit 11 supported by the engaging part 30C is also raised and lowered while maintaining the posture. Accordingly, the posture of the imaging unit 11 is stable even in a case of being raised or lowered.

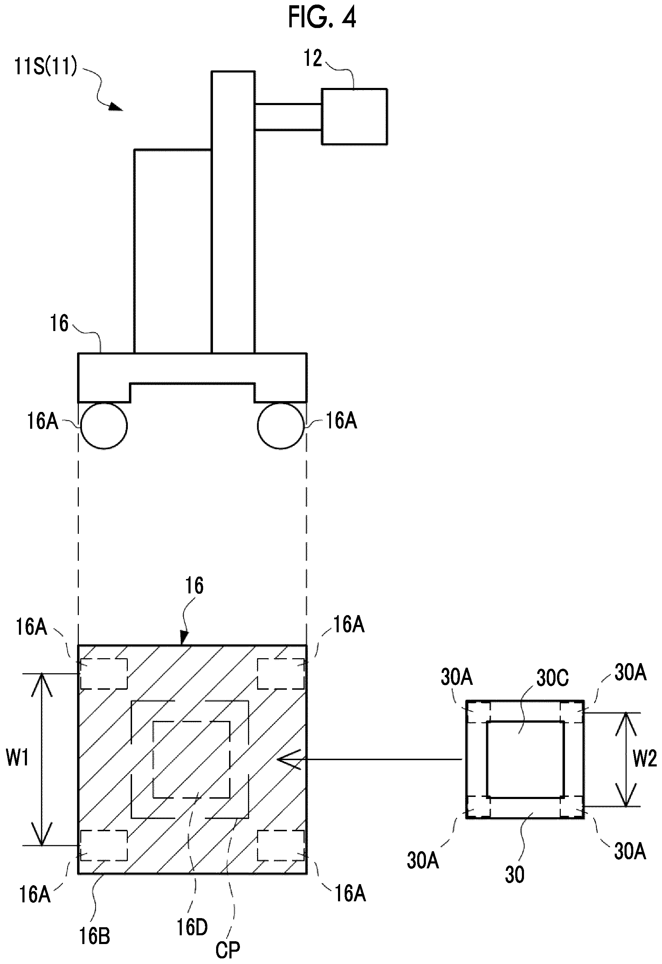

In addition, as shown in Fig. 4, at least a part of the electric traveling unit 30 enters a projection region of the imaging unit 11 in a case where the floor FL is used as a projection surface, in the combined state. In Fig. 4, a region shown by hatching is a projection region in which the floor FL of the carriage unit 16 of the imaging unit 11 is used as a projection surface. A reference sign CP indicates a combining position in a case where the electric traveling unit 30 combines with the imaging unit 11. In the present example, the entire electric traveling unit 30 enters the projection region indicated by hatching. Accordingly, in the combined state, an increase in the occupied space occupied by the imaging unit 11 is suppressed as compared with the non-combined state. In the present example, since the entire electric traveling unit 30 enters the projection region, the occupied space does not increase.

In addition, a wheel spacing W2 between a plurality of the wheels 30A of the electric traveling unit 30 is narrower than a wheel spacing W1 between the wheels 16A constituting the traveling mechanism of the imaging unit 11. Therefore, it is easy to reduce the size of the electric traveling unit 30.

Fig. 5 is a block diagram showing an electric configuration of the imaging unit 11 and the electric traveling unit 30. The radiation source unit 11S, which is an example of the imaging unit 11, comprises a processor 40, a storage 41, a communication interface (I/F) 42, an operation panel 43, and a battery 44.

The processor 40 functions as an irradiation controller that executes irradiation control of the irradiation unit 12 in addition to integrally controlling the entire radiation source unit 11S. As an example, the processor 40 is configured by a central processing unit (CPU) and a memory such as a random access memory (RAM), and functions as various controllers by executing a program loaded in the memory.

The storage 41 is a data storage that is composed of a hard disk drive, a solid state drive, a non-volatile memory, and the like and that stores data of various types of setting information in addition to the program.

The communication I/F 42 is, for example, a wireless communication unit, and executes wireless communication with another imaging unit 11 such as the panel unit 11D and wireless communication with the electric traveling unit 30.

The operation panel 43 is an operating part for inputting an operation instruction, and is composed of, for example, a touch panel type display. The battery 44 is a rechargeable battery and supplies power to each unit of the radiation source unit 11S.

The electric traveling unit 30 comprises a processor 50, a storage 51, a communication interface (I/F) 52, an actuator 53, a camera 54, and a battery 55.

The processor 50 functions as a traveling controller that integrally controls the entire electric traveling unit 30 and that mainly executes traveling control related to electric traveling. In addition, the processor 50 executes control of a combining position of the electric traveling unit 30 in a case of combining with the imaging unit 11, elevating control of the engaging part 30C, and the like. Similarly to the processor 40, the processor 50 is composed of, for example, a CPU and a memory such as a RAM, and functions as various controllers by executing a program loaded in the memory.

Similar to the storage 41, the storage 51 is also a data storage that is composed of a hard disk drive, a solid state drive, a non-volatile memory, and the like and that stores data of various types of setting information in addition to the program.

The communication I/F 52 is also, like the communication I/F 42, a wireless communication unit, for example, and executes wireless communication with the imaging unit 11. The setting information includes a home position, a registered position, and the like used for traveling control described later.

The actuator 53 has the motor M that drives the wheels 30A. The motor M includes a motor for causing the wheels 30A to travel and rotate and a motor for causing the wheels 30A to revolve. A measurement sensor MS measures a movement direction, a movement amount, and the like of the electric traveling unit 30, such as a rotation amount, a rotation direction, a revolution direction, and a revolution angle of the wheels 30A. A measurement value measured by the measurement sensor MS is input to the processor 50. The measurement sensor MS is composed of, for example, a rotary encoder and an acceleration sensor. The processor 50 executes the traveling control such as the self position estimation of the electric traveling unit 30 based on the received measurement value.

The camera 54 functions as a sensor used for executing control of automatically combining with the imaging unit 11. The camera 54 is, for example, an optical camera, and images the environment around the electric traveling unit 30 and inputs the captured camera image to the processor 50. For example, the cameras 54 are provided in plural on a side surface of the body part 30B, and the camera 54 is also provided on an upper surface. As will be described later, the processor 50 executes control of automatically combining with the imaging unit 11 based on the camera images of the plurality of the cameras 54.

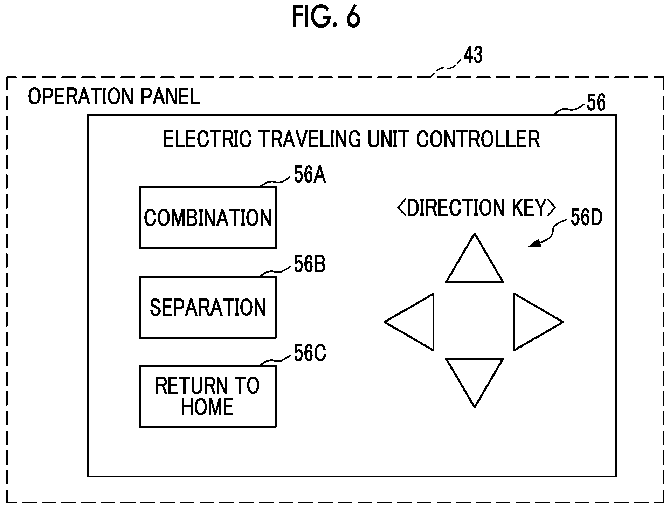

Fig. 6 is an example of a screen of a controller 56 for operating the electric traveling unit 30 displayed on the operation panel 43. The operation instruction including the movement instruction for the electric traveling unit 30 is input through the controller 56. The operation instruction input from the controller 56 is wirelessly transmitted from the communication I/F 42 to the communication I/F 52. As described above, the electric traveling unit 30 indirectly receives the operation instruction including the movement instruction via the imaging unit 11.

The controller 56 is provided with a combination button 56A, a separation button 56B, a return button 56C, and a direction key 56D. The combination button 56A is an operation button for inputting the combination instruction for the electric traveling unit 30 to automatically combine with the imaging unit 11 in a state of being separated from the imaging unit 11. The separation button 56B is an operation button for inputting a separation instruction for separating the electric traveling unit 30 from the imaging unit 11 in the combined state. The return button 56C is an operation button for inputting a return instruction for returning the electric traveling unit 30 to a home position HP set in advance as the initial position of the electric traveling unit 30. The direction key 56D is an operation button for inputting a movement instruction for moving in any one of four directions.

The combination button 56A, the separation button 56B, and the return button 56C are operation buttons for causing the electric traveling unit 30 to autonomously execute traveling control set in advance. On the other hand, the direction key 56D is an operation button for executing simple traveling control of only indicating a direction and moving the electric traveling unit 30 in the indicated direction according to the operation amount. The direction key 56D is a so-called deadman type in which the output of the operation signal is continued while the direction key 56D is being operated, and the output of the operation signal is stopped in a case where the operation is stopped. Therefore, the safety is higher than that in a case where the operation signal is output even in a state where the hand is released from the direction key 56D.

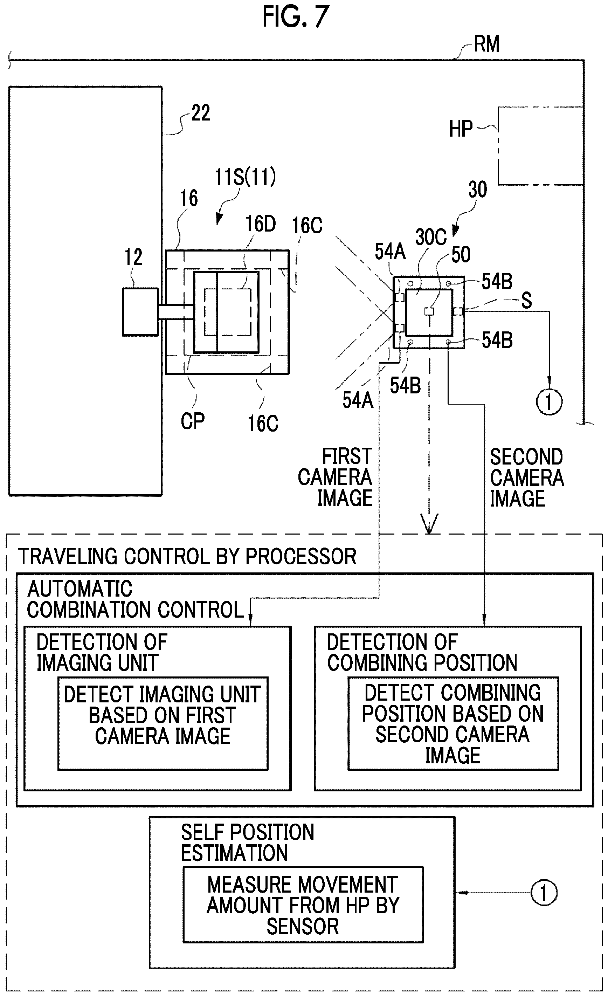

The traveling control of the electric traveling unit 30 executed by the processor 50 will be described with reference to Fig. 7. The autonomous traveling control executed by the electric traveling unit 30 is roughly divided into automatic combination control and self position estimation. The automatic combination control is the series of controls of detecting the position of the imaging unit 11, detecting a combining position CP, and bonding to the imaging unit 11.

In the electric traveling unit 30, in a case where the combination instruction is input by operating the combination button 56A, the processor 50 starts the detection of the imaging unit 11. In the detection of the imaging unit 11, the processor 50 acquires the first camera image from a first camera 54A on the side of the electric traveling unit 30. The processor 50 detects the imaging unit 11 in the periphery by, for example, a pattern matching method of collation between the first camera image and the form data of the imaging unit 11 set in advance. In a case where the imaging unit 11 is not present in the first camera image, the processor 50 causes the electric traveling unit 30 to travel randomly to search for the imaging unit 11. In addition, in a case where a plurality of the imaging units 11 are detected in the first camera image, for example, the processor 50 executes processing of presenting a plurality of candidates of the imaging units 11 to be combined on the operation panel 43 and causing the operator OP to designate the imaging unit 11 to be combined. In addition, in a case where the processor 50 detects the imaging unit 11, the detected imaging unit 11 may have already combined with another electric traveling unit 30. In that case, the processor 50 excludes the imaging unit 11 that has already combined with the other electric traveling unit 30 from the candidates for combination.

In addition, the first camera 54A is, for example, a stereo camera in which two optical systems are provided at intervals. The processor 50 can acquire depth information of the target to be imaged by using parallax of two first camera images formed by the two optical systems. Accordingly, the processor 50 can ascertain the distance of the detected imaging unit 11. A distance-measuring sensor may be used instead of the first camera 54A to ascertain the distance. For example, a laser imaging detection and ranging or light detection and ranging (LIDAR), a time-of-flight (TOF) camera, and the like can be applied as the distance-measuring sensor. In addition, a distance-measuring sensor using ultrasonic waves may be used.

In the electric traveling unit 30, in a case where the processor 50 detects the imaging unit 11, the processor 50 further detects the entry port 16C of the base unit 16B by a pattern matching method based on the first camera image. The electric traveling unit 30 travels toward the entry port 16C under the control of the processor 50. In a case where the electric traveling unit 30 reaches the entry port 16C, the electric traveling unit 30 enters below the base unit 16B through the entry port 16C while controlling the entry posture.

In the entry state, the processor 50 acquires the second camera image from a second camera 54B on the upper surface. The processor 50 detects the combining position CP of the electric traveling unit 30 based on the second camera image. Specifically, the processor 50 detects a marker (not shown) indicating the position of the recessed portion 16D by a pattern matching method based on the second camera image. The processor 50 adjusts the position of the electric traveling unit 30 according to the detected combining position CP such that the engaging part 30C and the recessed portion 16D face each other based on the position of the detected marker.

In addition, the processor 50 acquires the measurement value from the measurement sensor MS and ascertains, for example, the movement amount of the electric traveling unit 30 from the home position (indicated by HP in Fig. 7). The home position HP is, for example, a position registered as a reference position of the electric traveling unit 30 in an imaging room RM. As described above, the measurement sensor MS measures the measurement values such as the rotation amount, the rotation direction, the revolution amount, and the revolution angle of the wheels 30A. The processor 50 executes the self position estimation of estimating the relative self position of the electric traveling unit 30 with respect to the home position HP by accumulating these measurement values. Such self position estimation is self position estimation based on a so-called odometry method. By performing such self position estimation, it is possible to automatically return the electric traveling unit 30 to the home position HP. In addition to the home position HP, it is possible to perform the traveling control of automatically moving the electric traveling unit 30 to a registered position set in advance.

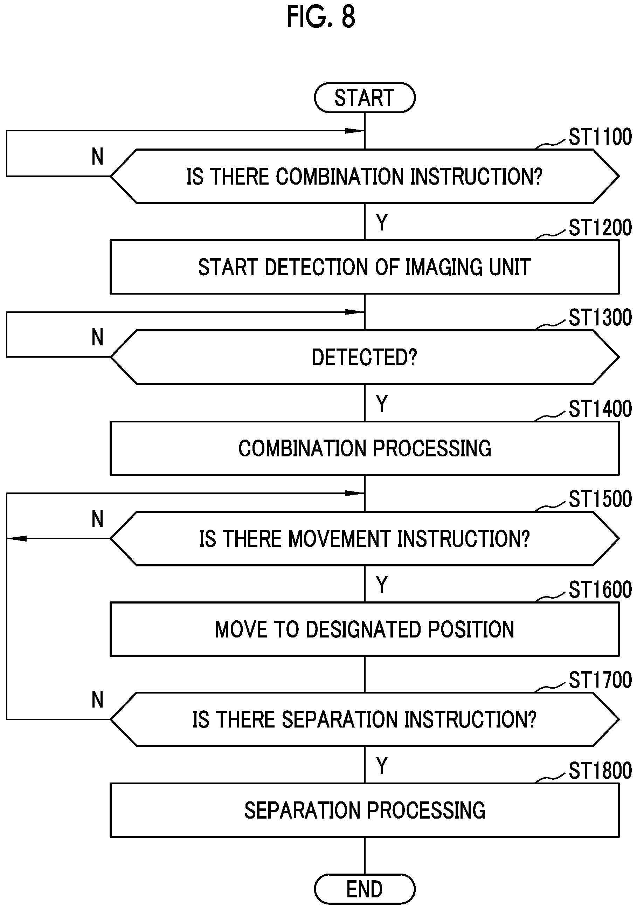

An operation of the above-mentioned configuration will be described with reference to a flowchart shown in Fig. 8. First, in a case in which the imaging is performed, the operator OP performs the registration of the imaging units 11 such as the radiation source unit 11S and the panel unit 11D, according to the imaging posture such as the upright posture and the decubitus posture, the imaging part such as the chest and the abdomen, and the like. The imaging unit 11 can also be moved manually. In a case where the operator OP manually performs the registration of the imaging unit 11, the imaging unit 11 is moved to a target location. The brake is applied so that the imaging unit 11 does not move inadvertently at the location to which the imaging unit 11 moved.

Meanwhile, in a case where the imaging unit 11 is moved by using the electric traveling unit 30, first, the operator OP operates the controller 56 of the operation panel 43 of the imaging unit 11 to combine the imaging unit 11 and the electric traveling unit 30 with each other.

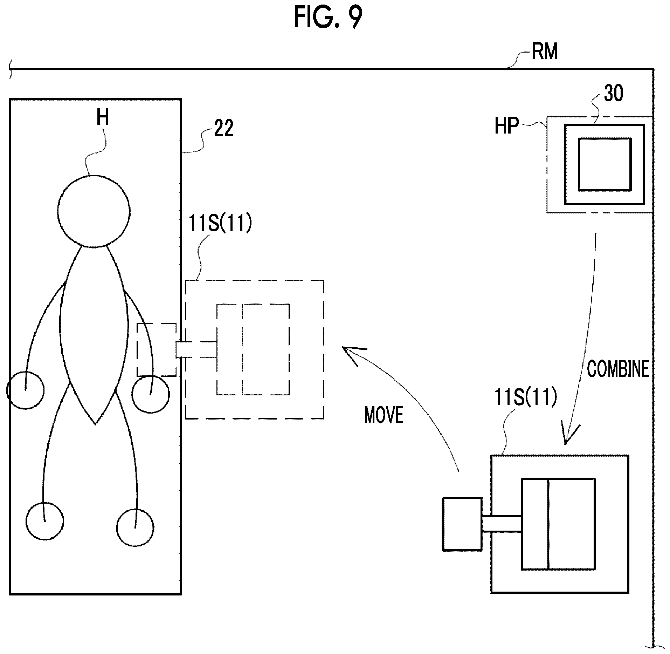

As an example, a case in which imaging is performed using the decubitus imaging table 22 and the radiation source unit 11S is considered as shown in Fig. 9. In the example shown in Fig. 9, the electric traveling unit 30 is at the home position HP, and the radiation source unit 11S is at a position away from the decubitus imaging table 22. The operator OP operates the combination button 56A of the controller 56 from the operation panel 43 of the radiation source unit 11S.

In step ST1100 shown in Fig. 7, the electric traveling unit 30 waits for an input of a combination instruction. In a case in which the combination instruction from the radiation source unit 11S, which is an example of the imaging unit 11, is received (Y in step ST1100), the process proceeds to step ST1200, and the electric traveling unit 30 starts the detection of the imaging unit 11. The electric traveling unit 30 acquires the first camera image from the first camera 54A. In a case where the imaging unit 11 is not detected from the first camera image, the electric traveling unit 30 starts traveling from the home position HP and searches for the imaging unit 11 while traveling randomly.

In step ST1300, in a case where the radiation source unit 11S is detected from the first camera image (Y in step ST1300), the electric traveling unit 30 proceeds to step ST1400 and starts the combination processing with the radiation source unit 11S.

In the combination processing of step ST1400, first, the electric traveling unit 30 travels toward the entry port 16C of the radiation source unit 11S and enters below the base unit 16B through the entry port 16C. Below the base unit 16B, the electric traveling unit 30 detects the combining position CP at which the recessed portion 16D of the base unit 16B and the engaging part 30C face each other from the second camera image, and adjusts the position. At the combining position CP, the electric traveling unit 30 moves the engaging part 30C to the rising position and engages the engaging part 30C with the recessed portion 16D. As a result, the radiation source unit 11S and the electric traveling unit 30 combine with each other. In the combined state, a load is generated above the radiation source unit 11S in the vertical direction, so that the traveling resistance of the radiation source unit 11S is reduced.

In step ST1500, in a case where the operator OP operates the direction key 56D of the controller 56, the movement instruction is input to the electric traveling unit 30 (Y in step ST1500). In step ST1600, the electric traveling unit 30 moves based on the input movement instruction. The operator OP moves the radiation source unit 11S toward the target position of the decubitus imaging table 22. Since the radiation source unit 11S travels electrically, the burden on the operator OP is less than that in a case of causing the radiation source unit 11S to travel manually.

In a case in which the imaging is ended and the electric traveling unit 30 is separated from the radiation source unit 11S, the operator OP operates the separation button 56B of the controller 56. In step ST1700, in a case where the separation button 56B is operated, a separation instruction is input to the electric traveling unit 30. In a case where the separation instruction is input, the electric traveling unit 30 proceeds to step ST1800 and executes the separation processing. In the separation processing, the electric traveling unit 30 returns the engaging part 30C to the lowering position to release the bonding. The base unit 16B is retracted from below. Further, in a case where the return button 56C of the controller 56 is operated, the electric traveling unit 30 moves to the home position HP registered in advance based on the self position estimation.

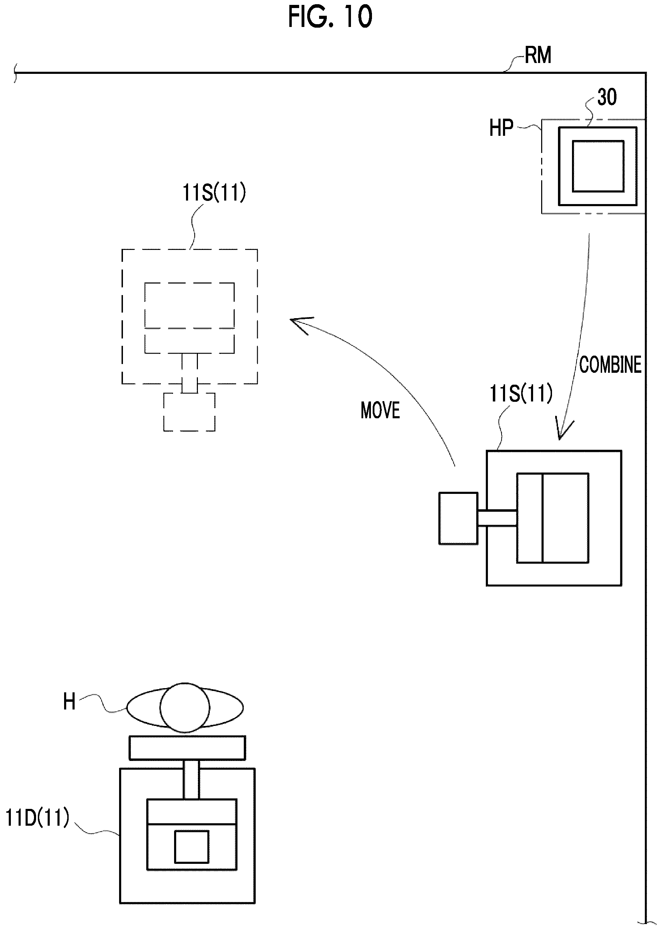

Fig. 10 is an example of a case in which upright imaging is performed. In this case, for example, after the electric traveling unit 30 is combined with the radiation source unit 11S, the operator OP moves the radiation source unit 11S by electric traveling toward a position facing the panel unit 11D on which the subject H is disposed.

As described above, the electric traveling unit 30 according to the technology of the present disclosure enables the electric traveling of the imaging unit 11 that includes the traveling mechanism traveling on the floor FL manually and the irradiation unit 12 (an example of a radiation source) or the detection panel 13 (an example of a radiation detector) used for radiography. The electric traveling unit 30 separably combines with the imaging unit 11, and reduces the traveling resistance of the traveling mechanism in the combined state than in the non-combined state. Since the traveling resistance is reduced, even in a case where the traveling resistance of the imaging unit 11 is large, the imaging unit 11 can travel electrically. For example, as in the above-described embodiment, even in a case where the traveling resistance is extremely large because the brake is applied to the imaging unit 11, the electric traveling unit 30 combines with the imaging unit 11 in a manner in which the traveling resistance is reduced. Therefore, even in a case where the traveling resistance of the imaging unit 11 is large, the imaging unit 11 can travel electrically.

Alternatively, even in a case where the traveling resistance due to friction with the floor FL is large due to the weight of the imaging unit 11, the traveling resistance is reduced by the combination, and thus the imaging unit 11 can travel electrically. In addition, since the traveling resistance is reduced, the driving force of the electric traveling unit 30 can be relatively small, which contributes to reduction in size.

As shown in Figs. 9 and 10, in the imaging, an operation of moving the imaging unit 11 is frequently performed in order to perform registration of the imaging unit 11. In some cases, manual traveling is convenient, but in some cases, electric traveling is convenient. In a case where the imaging unit 11 incorporates the motor for the electric traveling, there is a concern that the motor may be a load during manual traveling. By using the separable electric traveling unit 30, it is possible to use the manual traveling and the electric traveling together while taking advantage of the convenience of each.

In addition, in the above-described embodiment, in the electric traveling unit 30, a load is generated above the imaging unit 11 in the vertical direction in the combined state. Therefore, it is possible to reduce the frictional force generated between the imaging unit 11 and the floor FL with a relatively simple configuration. For example, in a case where the brake of the imaging unit 11 is applied, it is also possible to provide a mechanism for executing a brake release operation in the electric traveling unit 30 as a mechanism for reducing the traveling resistance in the combined state. However, since the mechanism for executing the brake release operation has a complicated configuration, it is better to generate the load upward in the vertical direction to be capable of realizing the reduction of the traveling resistance with a relatively simple configuration.

In addition, in the above-described embodiment, the electric traveling unit 30 has a load generating mechanism that generates a load, as shown using the elevating mechanism of the engaging part 30C as an example. Since a load is generated using a mechanism for generating the load, it is easy to control a force and a direction for generating the load as compared with a case where the mechanism is not used.

In addition, in the above-described embodiment, the load generating mechanism of the electric traveling unit 30 is composed of an elevating mechanism as shown in Fig. 3 using the engaging part 30C as an example. This elevating mechanism includes the engaging part 30C (an example of a support portion) that supports the imaging unit 11 while maintaining a posture of the imaging unit 11 in the non-combined state, and generates the load by raising and lowering the engaging part 30C. A load can be generated in a state in which the posture of the imaging unit 11 is stabilized in order to raise and lower the support portion of the imaging unit 11.

In addition, in the above embodiment, the load generated by the load generating mechanism is a load that lifts at least a part of the imaging unit 11 from the floor FL. By lifting the imaging unit 11 from the floor FL, the traveling resistance caused by friction can be reduced. Further, more specifically, as shown in Fig. 2, the load is a load for lifting the entire imaging unit 11 from the floor FL. By lifting the entire imaging unit 11, the traveling resistance caused by friction with the floor FL can be eliminated.

In addition, in the above-described embodiment, the imaging unit 11 is provided with a brake that increases the traveling resistance. In the above-described embodiment, as shown in Fig. 2 as an example, since the wheels 16A to which the brake is applied are lifted from the floor FL, the load generated by the load generating mechanism is a load that reduces the traveling resistance caused by the brake. As a result, the traveling resistance such as the rolling resistance of the wheels 16A due to the brake can be significantly reduced.

In addition, in the above-described embodiment, the electric traveling unit 30 mechanically bonds to the imaging unit 11 as shown using the engaging part 30C as an example. Such bonding suppresses the imaging unit 11 and the electric traveling unit 30 from being inadvertently separated, and the imaging unit 11 and the electric traveling unit 30 can combine with each other in a stable state.

In addition, in the above-described embodiment, at least a part of the electric traveling unit 30 enters a projection region of the imaging unit 11 in a case where the floor FL is used as a projection surface, in the combined state, as shown in Fig. 4 as an example. Therefore, even in the combined state, an increase in the occupied space of the imaging unit 11 is suppressed as compared with the non-combined state. In particular, in the above-described embodiment, in the combined state, the entire electric traveling unit 30 enters the projection region of the imaging unit 11. Therefore, the occupied space of the imaging unit 11 does not increase even in a case where the imaging unit 11 combines with the electric traveling unit 30. Of course, in the combined state, the entire electric traveling unit 30 may not enter the projection region.

In addition, in the above-described embodiment, the electric traveling unit 30 includes a plurality of wheels in which at least one wheel is a driving wheel that rotates electrically, and as shown in Fig. 4 as an example, the wheel spacing W2 between the plurality of wheels 30A is narrower than the wheel spacing W1 of the imaging unit 11. Accordingly, it is easy to reduce the size of the electric traveling unit. Since the imaging unit 11 is applied with an external force from various directions in the manual operation, the wider the wheel spacing W1, the more stable the imaging unit 11. On the other hand, since the direction in which the external force is applied in the combined state is almost determined, the electric traveling unit 30 is often allowed to have a narrow wheel spacing W2. Since the narrower the wheel spacing W2, the easier the size reduction, an increase in the occupied space is also suppressed as a result.

In addition, in the above-described embodiment, the electric traveling unit 30 has a function of automatically combining with the imaging unit 11 by a combination instruction in a state of being separated from the imaging unit 11, as shown in Fig. 7 as an example. This is convenient because the combination is automatically performed.

In addition, in the above-described embodiment, the electric traveling unit 30 detects a position of the imaging unit 11 and moves to the detected position. This is one of a so-called guide-less type autonomous traveling methods in which a traveling guide such as a guide passage or a guidance marker is not provided on the floor FL. Therefore, the electric traveling unit 30 is very effective in a case where the electric traveling unit 30 is used in the imaging room RM in which various devices are deployed and there are many obstacles to traveling. In addition, since the electric traveling unit 30 has a function of detecting the imaging unit 11, it is not necessary to register the movement destination, and thus the usability is favorable.

In addition, in the above-described embodiment, the electric traveling unit 30 has a function of automatically moving to a designated position such as the home position HP shown as an example. Since the electric traveling unit 30 automatically moves to the designated position registered in advance in this way, the usability is favorable.

In addition, in the above-described embodiment, the electric traveling unit 30 indirectly receives the operation instruction including the movement instruction via the imaging unit 11, as shown in Fig. 6 as an example. Accordingly, the usability is favorable as compared with a case where the imaging unit 11 and the electric traveling unit 30 are operated by separate operating parts. In addition, since the imaging unit 11 combines with the electric traveling unit 30, the electric traveling unit 30 can be operated through the imaging unit 11, which makes it very easy for the operator OP to use the imaging unit 11.

In addition, in the above-described embodiment, the electric traveling unit 30 includes a wireless communication unit that performs wireless communication with the imaging unit 11. Accordingly, the operation instruction from the imaging unit 11 can be wirelessly received. Since the electric traveling unit 30 repeatedly combines with and separates from the imaging unit 11, the usability is most favorable for wireless connection.

In addition, in the above-described embodiment, the electric traveling unit 30 receives the movement instruction as the operation instruction through an operating part of a type in which an output of an operation signal is continued while the operating part is being operated and the output of the operation signal is stopped in a case where the operation is stopped, as shown using the direction key 56D shown in Fig. 6 as an example. Since this is a so-called deadman type operating part, the inadvertent travel of the electric traveling unit 30 is suppressed, and thus the safety is high.

In addition, the electric traveling unit 30 according to the technology of the present disclosure can be variously modified as described below.

### Modification Example 1: registering movement destination

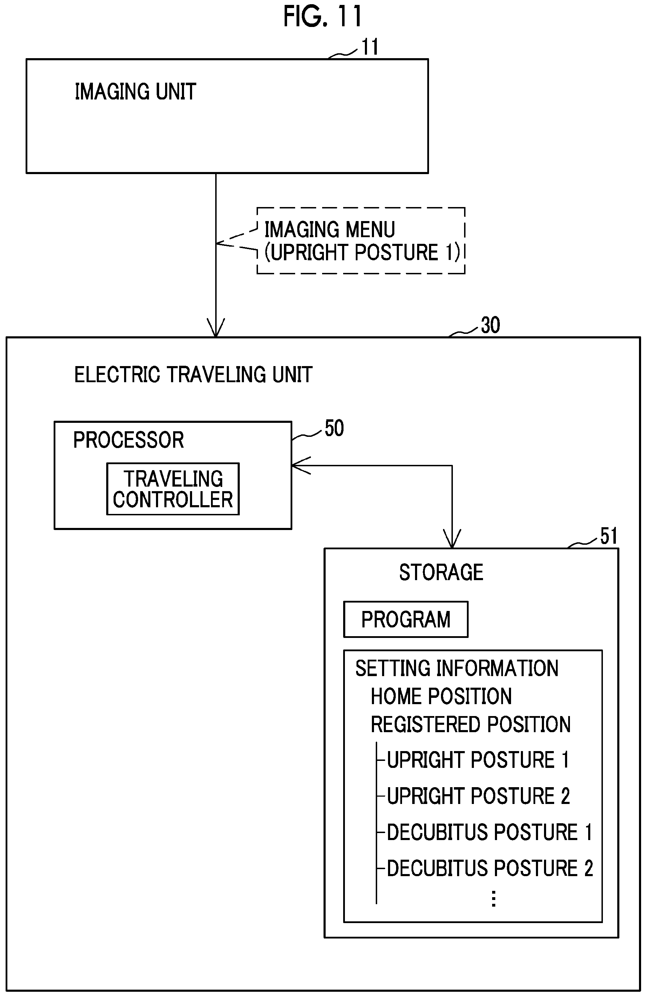

In the above-described embodiment, an example in which the electric traveling unit 30 automatically moves to the designated position has been described using the home position HP as an example of the position registered in advance, but a position other than the home position HP may be used as the registered position. For example, as shown in Modification Example 1 of Fig. 11, the registered position may be registered in association with the imaging menu, and the movement destination may be set to the corresponding registered position in a case where the imaging menu is selected.

As shown in Fig. 11, as the registered position, for example, as the registered position corresponding to the upright imaging, positions such as an upright posture 1 and an upright posture 2 are registered, and as the registered position corresponding to the decubitus imaging, positions such as a decubitus posture 1 and a decubitus posture 2 are registered. The registered positions are positions at which the radiation source unit 11S or the panel unit 11D is set according to the imaging menu, and are registered in association with the imaging menu. These pieces of information are stored in the storage 51 of the electric traveling unit 30.

In a case where an imaging menu that defines the imaging technique is selected on the operation panel 43 of the imaging unit 11, the processor 50 of the electric traveling unit 30 acquires, for example, an imaging menu corresponding to the decubitus posture 1 as the selected imaging menu. The processor 50 sets the registered position of the decubitus posture 1 corresponding to the imaging menu as the movement destination of the electric traveling unit 30. The processor 50 moves the electric traveling unit 30 toward the set movement destination. As the information on each registered position, for example, a movement amount (including a direction) with respect to the home position HP is set. The processor 50 calculates the movement amount to the registered position by using the self position estimation function based on the home position HP and performs the traveling control to the registered position.

A mark visible to the operator OP may be attached to the floor FL or the like so that the operator OP can accurately set the position of the imaging unit 11 in advance at the registered position that is the movement destination of the electric traveling unit 30. In addition, in a case in which the decubitus imaging table 22 is used, a bonding position at which the decubitus imaging table 22 and the imaging unit 11 are bonded to each other may be set in advance, and the position may be set as the registered position.

### Modification Example 2: part is exposed in combined state

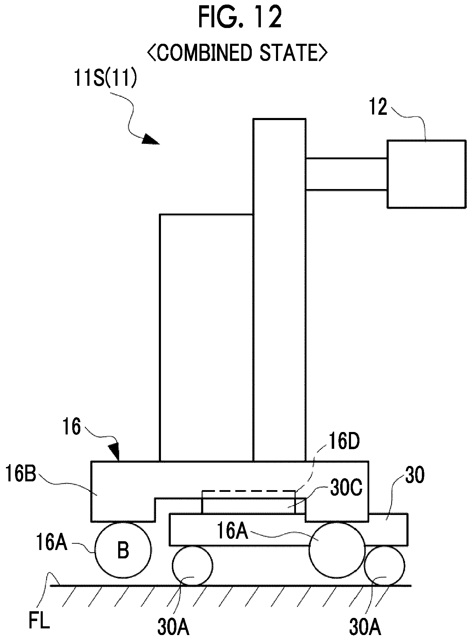

In the above-described embodiment, as shown in Fig. 4, an example in which the entire electric traveling unit 30 enters the projection region of the imaging unit 11 in the combined state has been described. However, as shown in Modification Example 2 of Fig. 12, in the combined state, an aspect in which a part of the electric traveling unit 30 enters the projection region may be adopted.

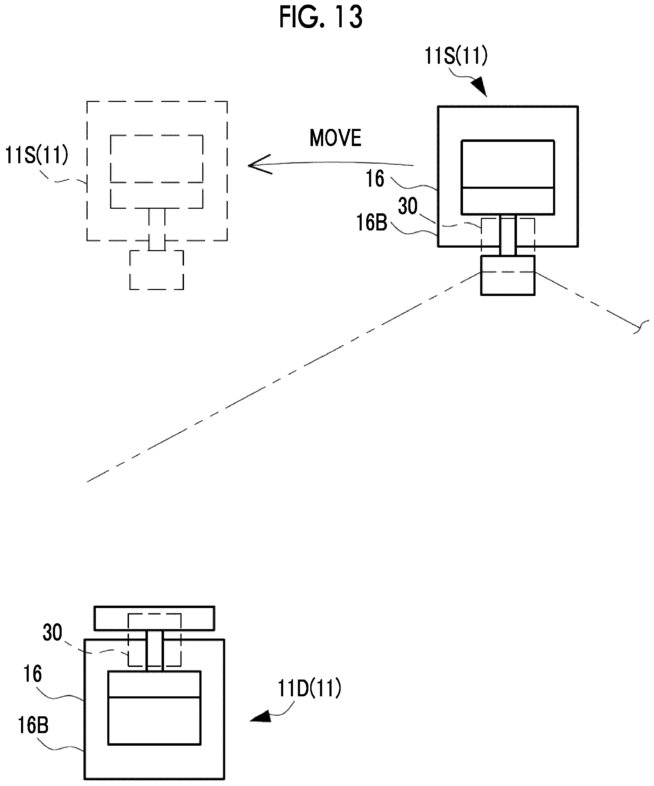

In this case, a part of the electric traveling unit 30 is exposed to the outside of the entry port 16C. This has the following advantages. For example, as shown in Fig. 13, a case where one electric traveling unit 30 detects another electric traveling unit 30 using the first camera 54A (see Fig. 7) and performs registration in accordance with the detected electric traveling unit 30 is considered. More specifically, one electric traveling unit 30 combines with the radiation source unit 11S, and the other electric traveling unit 30 combines with the panel unit 11D. The electric traveling unit 30 combined with the radiation source unit 11S detects the electric traveling unit 30 combined with the panel unit 11D, and performs the registration of the radiation source unit 11S in accordance with the position of the panel unit 11D. In this case, in a case where a part of the detection target electric traveling unit 30 that combines with the panel unit 11D is exposed outward from the base unit 16B of the panel unit 11D, the electric traveling unit 30 of the panel unit 11D is easily detected from the electric traveling unit 30 of the radiation source unit 11S. In this way, there is a case where it is advantageous that a part of the electric traveling unit 30 is exposed from the projection region of the imaging unit 11.

### Modification Example 3: grounded state in combined state

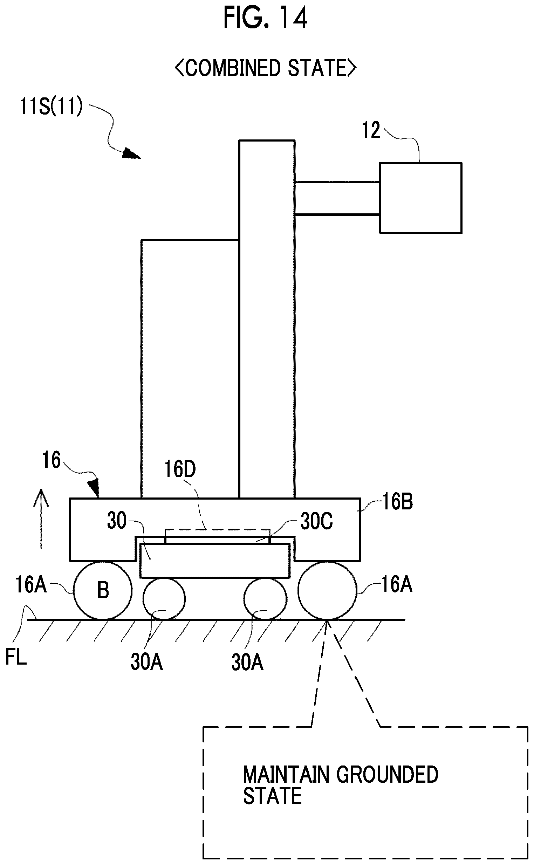

In the above-described embodiment, as shown in Fig. 2, in the combined state, the imaging unit 11 is lifted from the floor FL by generating a load above the imaging unit 11 in the vertical direction. However, as shown in Fig. 14, the grounded state of the imaging unit 11 may be maintained while a load is generated upward in the vertical direction. That is, a portion of the imaging unit 11 that is grounded on the floor FL in the non-combined state is maintained in the grounded state even in a state where the load is generated. For example, an elastic member such as rubber is used for the wheels 16A, and the wheels 16A are elastically deformed by the weight of the imaging unit 11. In a case where a load is generated upward in the vertical direction by the electric traveling unit 30, a part of the weight of the imaging unit 11 applied to the wheels 16A is reduced. The load upward in the vertical direction reduces the deformation amount of the wheels 16A, but is set to a load to the extent that the grounded state of the wheels 16A is maintained. Accordingly, the grounded state of the wheels 16A is maintained. As described above, since the load is generated upward in the vertical direction even in a case where the grounded state is maintained, the frictional force between the wheels 16A of the imaging unit 11 and the floor FL is reduced, and thus the traveling resistance is reduced. In addition, since the grounded state of the imaging unit 11 is maintained, there is also an effect that the posture of the imaging unit 11 can be stabilized.

### Modification Example 4: aspect in which load generating mechanism is not provided

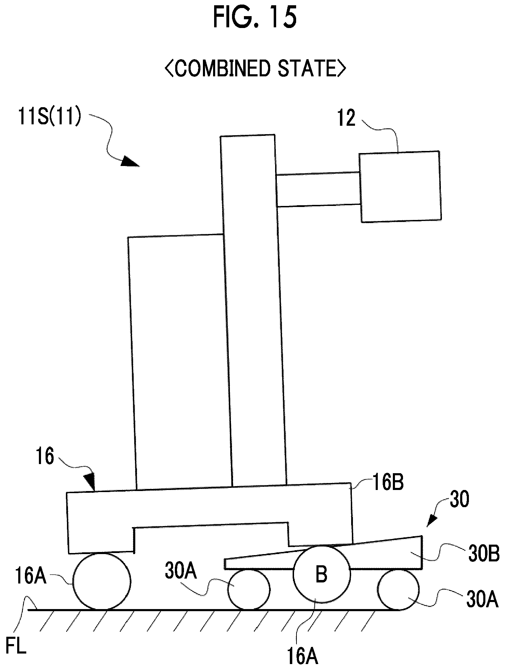

In the above-described embodiment, the example has been described in which the load generating mechanism such as the elevating mechanism that raises and lowers the engaging part 30C as shown in Fig. 3 is provided, but the load can also be generated without providing the load generating mechanism such as the elevating mechanism as shown in Fig. 15. The example shown in Fig. 15 is an example in which the shape of the body part 30B of the electric traveling unit 30 is a wedge shape. In this case, in a case where a part of the electric traveling unit 30 enters below the base unit 16B of the imaging unit 11, the upper surface of the body part 30B touches the bottom portion of the base unit 16B, and thus a load is generated above the imaging unit 11 in the vertical direction. In this state, the electric traveling unit 30 combines with the imaging unit 11 at an engaging part (not shown). Since a load is generated upward in the vertical direction in the combined state, the traveling resistance of the imaging unit 11 is reduced. In the example shown in Fig. 15, the wheel 16A to which the brake is applied is lifted among the wheels 16A of the base unit 16B.

As is clear in the above-described embodiment, the load generated "upward in the vertical direction" according to the technology of the present disclosure includes a case where a part of the components of the load applied to the imaging unit 11 is directed upward in the vertical direction by generating a load in an oblique direction with respect to the vertical direction as shown in Fig. 15, in addition to a case where all the components are directed upward in the vertical direction as shown in Fig. 2.

### Modification Example 5: guide unit to combining position

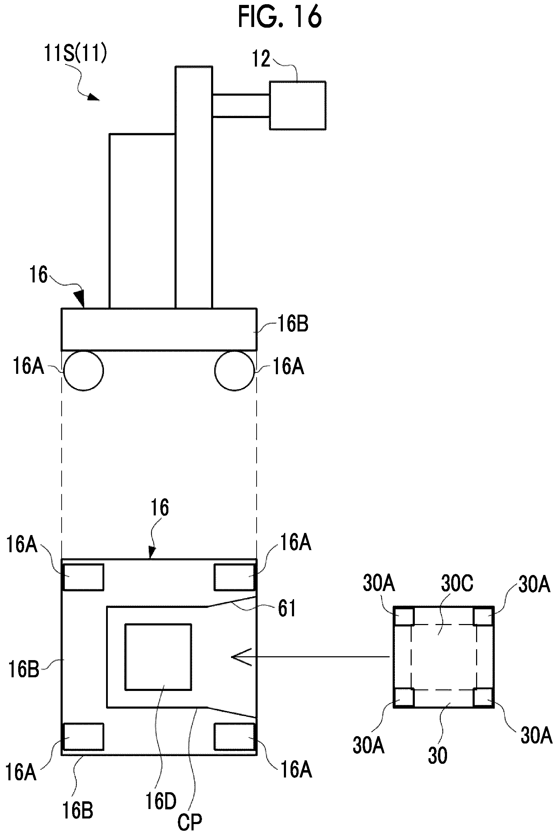

In the above-described embodiment, as shown in Fig. 7, an example in which the second camera 54B detects the combining position CP below the base unit 16B of the imaging unit 11 has been described. However, as shown in Fig. 16, a guide unit 61 may be provided in the bottom portion of the base unit 16B. In Fig. 16, unlike Fig. 7, the base unit 16B and the electric traveling unit 30 are shown in a state of being viewed in a plan view from below. The guide unit 61 touches the body part 30B of the electric traveling unit 30 to guide the electric traveling unit 30 to the combining position CP. Accordingly, the second camera 54B is also unnecessary, and the control of the registration can be easily performed.

### Modification Example 6: auxiliary battery

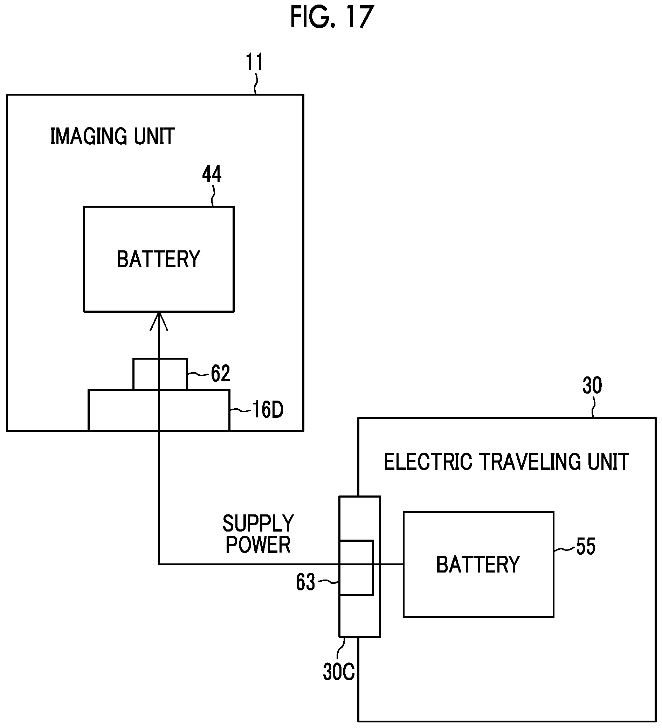

In addition, as shown in Fig. 17, the battery 55 of the electric traveling unit 30 may be caused to function as an auxiliary battery of the imaging unit 11 in the combined state. In this case, a connector 63 having an electrical contact for supplying power to the battery 44 of the imaging unit 11 is provided in the electric traveling unit 30. The connector 63 is provided in, for example, the engaging part 30C, and in a case where the engaging part 30C is engaged with the recessed portion 16D, the connector 63 is electrically connected to a connector 62 provided in the recessed portion 16D. Accordingly, the power of the battery 55 of the electric traveling unit 30 is supplied to the battery 44 of the imaging unit 11. Accordingly, the battery 55 functions as the auxiliary battery of the imaging unit 11. A dedicated battery used as an auxiliary battery may be provided in the electric traveling unit 30 in addition to the battery 55.

### Other Modification Examples

In addition, the bonding between the electric traveling unit 30 and the imaging unit 11 has been described using the engagement between the engaging part 30C and the recessed portion 16D as an example, but instead of or in addition to these, a bonding pin or the like may be provided. The bonding is performed with the bonding pin, so that the bonding can be more firmly performed. As the bonding pin, for example, a plunger of a solenoid can be used.

In addition, the bonding of the electric traveling unit 30 and the imaging unit 11 may not be mechanical bonding by using the engaging part 30C or the like; for example, the electric traveling unit 30 and the imaging unit 11 may be bonded to each other by an electromagnet. In addition, the connector 63 for power supply shown in Fig. 17 may not be a contact-type connector and may be a noncontact-type connector capable of noncontact power supply.

In addition, although the example in which the electric traveling unit 30 is wirelessly connected to the imaging unit 11 has been described, the electric traveling unit 30 may be connected to the imaging unit 11 in a wired manner. However, in the case of the wired connection, it is necessary to use the remote controller for the electric traveling unit 30 for at least the operation instruction until the electric traveling unit 30 combines with the imaging unit 11. In addition, the wired connection may be performed using the connector 62, the connector 63, and the like shown in Fig. 17, or a communication cable may be used. Since the wired connection is time-consuming in this way, the wireless connection is preferable.

In addition, the operation instruction of the electric traveling unit 30 may be performed by voice input.

In addition, for the autonomous traveling control of the electric traveling unit 30, a simultaneous localization and mapping (SLAM) technique of performing autonomous traveling while simultaneously performing self position estimation and map creation may be used.

The forms of the wheels shown in the above-described embodiment are merely examples, and can be modified in various ways. For example, the number of front wheels and/or the number of rear wheels may be independently changed. For example, one front wheel and two rear wheels may be combined or two front wheels and three rear wheels may be combined. Further, for example, the sizes and shapes of the front wheels and the rear wheels may be changed.

In addition, in the above-described embodiment, various types of hardware described below can be used as the hardware of the processor 50. The various types of hardware include, in addition to a CPU that is a general-purpose processor that executes software (program) to function as various processing units, a programmable logic device (PLD) of which a circuit configuration can be changed after manufacturing, such as a field-programmable gate array (FPGA), and a dedicated electric circuit that is hardware having a circuit configuration dedicatedly designed for executing specific processing, such as an application specific integrated circuit (ASIC).

Various types of processing described above may be executed by one of the various types of hardware or may be executed by a combination of two or more types of hardware (for example, a combination of a plurality of FPGAs or a CPU and an FPGA) of the same type or different types. In addition, a plurality of the processing units may be configured by one hardware. As an example in which the plurality of processing units are configured by one hardware, there is a form in which hardware that realizes all functions of a system including the plurality of processing units by using one integrated circuit (IC) chip is used, such as a system on a chip (SOC).

In this way, the various processing units are configured by using one or more of the various types of hardware described above.

Furthermore, as the various hardware structures, more specifically, an electrical circuit (circuitry) in which circuit elements such as semiconductor elements are combined can be used.

In addition, the technology of the present disclosure extends to a program for operating the electric traveling unit 30; as well as a computer-readable storage medium that non-temporarily stores the program (such as universal serial bus (USB) memory or digital versatile disc (DVD)-read only memory (ROM)). In addition, the technology of the present disclosure can also be applied to a program and a program product.

The content of the above description and the content of the drawings are detailed description of portions according to the technology of the present disclosure, and are merely examples of the technology of the present disclosure. For example, the above description relating to configurations, functions, operations, and advantageous effects is description relating to an example of configurations, functions, operations, and advantageous effects of the portions according to the technology of the present disclosure. Thus, it is needless to say that unnecessary portions may be deleted, new elements may be added, or replacement may be made to the content of the above description and the content of the drawings without departing from the gist of the technology of the present disclosure. Furthermore, to avoid confusion and to facilitate understanding of the portions according to the technology of the present disclosure, description relating to common technical knowledge and the like that does not require particular description to enable implementation of the technology of the present disclosure is omitted from the content of the above description and from the content of the drawings.

The technologies according to the following supplementary notes can be understood from the above description.

### Supplementary Note 1

An electric traveling unit that enables electric traveling of an imaging unit that includes a traveling mechanism traveling on a floor manually and a radiation source or a radiation detector used for radiography, in which the electric traveling unit separably combines with the imaging unit, and reduces a traveling resistance of the traveling mechanism in a combined state than in a non-combined state.

### Supplementary Note 2

The electric traveling unit according to Supplementary Note 1, in which a load is generated above the imaging unit in a vertical direction in the combined state.

### Supplementary Note 3

The electric traveling unit according to Supplementary Note 2, comprising: a load generating mechanism that generates the load.

### Supplementary Note 4

The electric traveling unit according to Supplementary Note 3, in which the load generating mechanism includes a support portion that supports the imaging unit while maintaining a posture of the imaging unit in the non-combined state, and generates the load by raising and lowering the support portion.

### Supplementary Note 5

The electric traveling unit according to any one of Supplementary Notes 2 to 4, in which the load is a load that lifts at least a part of the imaging unit from the floor.

### Supplementary Note 6

The electric traveling unit according to any one of Supplementary Notes 3 to 5, in which the imaging unit is provided with a brake that increases the traveling resistance, and the load is a load that reduces the traveling resistance caused by the brake.

### Supplementary Note 7

The electric traveling unit according to any one of Supplementary Notes 2 to 6, in which a portion of the imaging unit that is grounded on the floor in the non-combined state is maintained in a grounded state even in a state where the load is generated.

### Supplementary Note 8

The electric traveling unit according to any one of Supplementary Notes 1 to 7, in which the electric traveling unit mechanically or magnetically bonds to the imaging unit.

### Supplementary Note 9

The electric traveling unit according to any one of Supplementary Notes 1 to 8, in which at least a part of the electric traveling unit enters a projection region of the imaging unit in a case where the floor is used as a projection surface, in the combined state.

### Supplementary Note 10

The electric traveling unit according to Supplementary Note 9, comprising: a plurality of wheels in which at least one wheel is a driving wheel that rotates electrically, in which a wheel spacing between the plurality of wheels is narrower than a wheel spacing of the imaging unit.

### Supplementary Note 11

The electric traveling unit according to Supplementary Note 9 or 10, in which an entire electric traveling unit enters the projection region of the imaging unit in a case where the floor is used as the projection surface, in the combined state.

### Supplementary Note 12

The electric traveling unit according to any one of Supplementary Notes 1 to 11, in which the electric traveling unit has a function of automatically combining with the imaging unit by a combination instruction in a state of being separated from the imaging unit.

### Supplementary Note 13

The electric traveling unit according to Supplementary Note 12, in which the electric traveling unit detects a position of the imaging unit and moves to the detected position.

### Supplementary Note 14

The electric traveling unit according to any one of Supplementary Notes 1 to 13, in which the electric traveling unit has a function of automatically moving to a designated position.

### Supplementary Note 15

The electric traveling unit according to any one of Supplementary Notes 1 to 14, in which the electric traveling unit indirectly receives an operation instruction including a movement instruction via the imaging unit.

### Supplementary Note 16

The electric traveling unit according to Supplementary Note 15, comprising: a wireless communication unit that performs wireless communication with the imaging unit.

### Supplementary Note 17

The electric traveling unit according to Supplementary Note 15 or 16, in which the electric traveling unit receives the movement instruction as the operation instruction through an operating part of a type in which an output of an operation signal is continued while the operating part is being operated and the output of the operation signal is stopped in a case where the operation is stopped.

### Supplementary note 18

The electric traveling unit according to any one of Supplementary Notes 1 to 17, comprising: a battery that functions as an auxiliary battery of the imaging unit in the combined state.

### Supplementary note 19

A radiography system comprising: at least one imaging unit that includes a traveling mechanism traveling on a floor manually and a radiation source or a radiation detector used for radiography; and an electric traveling unit that enables electric traveling of the imaging unit, the electric traveling unit separably combining with the imaging unit and reducing a traveling resistance of the traveling mechanism in a combined state than in a non-combined state.

In this specification, "A and/or B" is synonymous with "at least one of A or B". That is, "A and/or B" may refer to A alone, B alone, or a combination of A and B. Furthermore, in this specification, a similar concept to "A and/or B" applies to a case in which three or more matters are expressed by linking the matters with "and/or".

All cited documents, patent applications, and technical standards described in the specification are incorporated by reference in the specification to the same extent as in a case where each individual cited document, patent application, or technical standard is specifically and individually indicated to be incorporated by reference.

## Claims

1. An electric traveling unit (30) that enables electric traveling of an imaging unit (11) that includes a traveling mechanism traveling on a floor (FL) manually and a radiation source or a radiation detector used for radiography,
wherein the electric traveling unit (30) separably combines with the imaging unit (11), and reduces a traveling resistance of the traveling mechanism in a combined state than in a non-combined state.

2. The electric traveling unit (30) according to claim 1,
wherein a load is generated above the imaging unit (11) in a vertical direction in the combined state.

3. The electric traveling unit (30) according to claim 2, comprising:
a load generating mechanism that generates the load.

4. The electric traveling unit (30) according to claim 3,
wherein the load generating mechanism includes a support portion that supports the imaging unit (11) while maintaining a posture of the imaging unit (11) in the non-combined state, and generates the load by raising and lowering the support portion.

5. The electric traveling unit (30) according to claim 2,
wherein the load is a load that lifts at least a part of the imaging unit (11) from the floor (FL).

6. The electric traveling unit (30) according to claim 3,
wherein the imaging unit (11) is provided with a brake that increases the traveling resistance, and
the load is a load that reduces the traveling resistance caused by the brake.

7. The electric traveling unit (30) according to claim 2,
wherein a portion of the imaging unit (11) that is grounded on the floor (FL) in the non-combined state is maintained in a grounded state even in a state where the load is generated.

8. The electric traveling unit (30) according to claim 1,
wherein the electric traveling unit mechanically or magnetically bonds to the imaging unit (11).

9. The electric traveling unit (30) according to claim 1,
wherein at least a part of the electric traveling unit (30) enters a projection region of the imaging unit (11) in a case where the floor (FL) is used as a projection surface, in the combined state.

10. The electric traveling unit (30) according to claim 9, comprising:
a plurality of wheels (30A) in which at least one wheel is a driving wheel that rotates electrically,
wherein a wheel spacing between the plurality of wheels (30A) is narrower than a wheel spacing of the imaging unit (11).

11. The electric traveling unit (30) according to claim 9,
wherein an entire electric traveling unit enters the projection region of the imaging unit (11) in a case where the floor (FL) is used as the projection surface, in the combined state.

12. The electric traveling unit (30) according to claim 1,
wherein the electric traveling unit (30) has a function of automatically combining with the imaging unit (11) by a combination instruction in a state of being separated from the imaging unit (11).

13. The electric traveling unit (30) according to claim 12,
wherein the electric traveling unit (30) detects a position of the imaging unit (11) and moves to the detected position.

14. The electric traveling unit (30) according to claim 1,
wherein the electric traveling unit (30) has a function of automatically moving to a designated position.

15. The electric traveling unit (30) according to claim 1,
wherein the electric traveling unit indirectly receives an operation instruction including a movement instruction via the imaging unit (11).

16. The electric traveling unit (30) according to claim 15, comprising:
a wireless communication unit that performs wireless communication with the imaging unit (11).

17. The electric traveling unit (30) according to claim 15,
wherein the electric traveling unit (30) receives the movement instruction as the operation instruction through an operating part of a type in which an output of an operation signal is continued while the operating part is being operated and the output of the operation signal is stopped in a case where the operation is stopped.

18. The electric traveling unit (30) according to claim 1, comprising:
a battery that functions as an auxiliary battery of the imaging unit (11) in the combined state.

19. A radiography system comprising:
at least one imaging unit (11) that includes a traveling mechanism traveling on a floor (FL) manually and a radiation source or a radiation detector used for radiography; and
an electric traveling unit (30) that enables electric traveling of the imaging unit (11), the electric traveling unit (30) separably combining with the imaging unit (11) and reducing a traveling resistance of the traveling mechanism in a combined state than in a non-combined state.
